# EUROPEAN PATENT APPLICATION

(11) **EP 4 817 626 A1**
(43) Date of publication of application: **07.10.2026**
(21) Application number: 24896640.0
(22) Date of filing: 28.11.2024
(51) Int. Cl.: A61K 39/25, A61K 39/39, A61P 31/22

(54) **VARICELLA-ZOSTER VACCINE COMPOSITION AND USE THEREOF**

(30) Priority: 30.11.2023 CN 202311615561
(71) Applicant: Huapu Shijiazhuang Pharmaceutical Co., Ltd., Shijiazhuang, Hebei 050035 (CN)
(72) Inventor: WANG, Xinzhong, Shijiazhuang, Hebei 050035 (CN); ZENG, Ting, Shijiazhuang, Hebei 050035 (CN)
(74) Representative: Witte, Weller & Partner Patentanwälte mbB
(86) International application number: PCT/CN2024/135229
(87) International publication number: WO 2025/113554

(57) **Abstract**

A varicella-zoster vaccine composition and use thereof, belonging to the technical field of biological vaccines. The composition comprises a VZV gE antigen and a CpG ODN adjuvant and further comprises an aluminum adjuvant. The nucleotide sequence of the CpG ODN adjuvant is set forth in SEQ ID NO: 1. The CpG ODN adjuvant with a specific nucleotide sequence has better immunostimulatory activity. The provided varicella-zoster vaccine composition can generate a higher humoral immune response earlier and induce protective immunity in advance for at least one month. One immunization can reach or be close to the antibody level of two immunizations of the Shingrix vaccine. Moreover, the longer-time stable high-humoral immunity level can be kept, and cellular immunity can also be generated.

## Description

The present application claims priority to Chinese Application No. 202311615561.8, filed on November 30, 2023 with the China Patent Office, entitled "Varicella-Zoster Vaccine Composition and Use Thereof," the entire contents of which are incorporated herein by reference.

### FIELD OF THE INVENTION

The present application relates to the field of biological vaccine technology, and more particularly to a varicella-zoster vaccine composition and use thereof.

### BACKGROUND OF THE INVENTION

Varicella-zoster virus (VZV), or human herpesvirus 3, is a neurotropic human alphaherpesvirus that causes varicella and herpes zoster (HZ). Herpes zoster (HZ) is an acute dermatological disease caused by the reactivation of latent varicella-zoster virus (VZV) in the body, primarily occurring in immunocompromised populations such as the elderly. In China, approximately 1.56 million new cases of herpes zoster are diagnosed annually among the population aged 50 and older; both the risk of incidence and the severity of the condition increase with age.

Herpes zoster is a self-limiting disease, clinically characterized primarily by a band-like patchy rash. Postherpetic neuralgia (PHN) is a chronic pain disorder and constitutes one of the most common complications of the disease. Herpes zoster vasculitis is associated with morbidity and mortality; furthermore, renal and gastrointestinal complications have also been reported. The primary treatment involves early intervention with acyclovir or brivudine. Vaccination of the elderly and immunocompromised patients is the main strategy for the prevention and control of herpes zoster.

Currently, four herpes zoster vaccines have been approved for marketing globally: Zostavax by Merck & Co., Inc. (MSD), SkyZoster by SK bioscience (a subsidiary of SK Chemicals), Ganwei by BCHT Pharm, and Shingrix by GlaxoSmithKline (GSK).

Merck's Zostavax is a live attenuated vaccine primarily indicated for individuals aged 50 and older; however, its efficacy declines as age increases. Furthermore, it is generally unsuitable for populations receiving immunosuppressive therapy or those with underlying immune disorders, such as HIV-infected individuals, due to the residual viral activity in live attenuated vaccines. There are similar issues for the SkyZoster vaccine and BCHT Pharm's Ganwei, as they are also live attenuated vaccines.

To address the limitations regarding the eligible population for live-attenuated herpes zoster vaccines, further research and development efforts have led to the creation of inactivated and recombinant protein vaccines. Recombinant protein vaccines are produced using genetic engineering techniques to generate immunologically active proteins or peptide fragments, which serve as the active ingredients of the vaccine. However, compared to live-attenuated herpes zoster vaccines, both inactivated and recombinant protein vaccines exhibit lower immunogenicity, resulting in relatively lower vaccine efficacy.

To enhance vaccine immunogenicity, it has been found that adjuvants play a crucial role in both the prevention and treatment of herpes zoster. Developing a vaccine by selecting an appropriate adjuvant-thereby boosting cellular and humoral immune responses within the target population-can effectively prevent herpes zoster. Aluminum adjuvants can induce immune enhancement and facilitate macrophages in establishing a memory immune response. For example, LZ901, a recombinant herpes zoster vaccine developed by Beijing Luzhu Biotechnology Co., Ltd., contains recombinant herpes zoster virus gE protein and an aluminum adjuvant with a single dose of 0.5 mL.

To further enhance the immunogenicity of vaccines, new adjuvants have been progressively developed. For instance, GlaxoSmithKline's Shingrix-a recombinant zoster vaccine (CHO cells)-utilizes the novel adjuvant system AS01_{B} (comprising MPL and QS-21). CpG ODN adjuvants represent a class of novel immunostimulants discovered in recent years; chemically, they are oligodeoxynucleotides containing unmethylated cytosine-phosphate-guanine (CpG) motifs. They elicit immune responses analogous to those triggered by natural CpG motifs through pattern recognition receptors; specifically, they bind to Toll-like receptors and effectively trigger mammalian immune responses via the TLR9 signaling pathway. The immune response elicited by CpG ODN adjuvants is predominantly Th1-type; it can induce a shift from a Th2-type to a Th1-type immune response, thereby stimulating cellular immunity. By activating immunocompetent cells-such as T cells, B cells, and NK cells-and inducing the production of a wide array of cytokines, it enhances both the specific and non-specific immune effects of the organism, serving as a vital link connecting innate and adaptive immunity.

Given the limited selection of currently available herpes zoster vaccines, there is an urgent need in the art to develop new VZV vaccine products to meet the vaccination needs of the expanding aging population. Consequently, it is a highly challenging technical strategy to develop novel vaccines, particularly through the exploration of novel adjuvants or formulation combinations to enhance the level of vaccine-induced immune responses.

### SUMMARY OF THE INVENTION

In view of the above, the primary objective of the present disclosure is to provide a varicella-zoster virus (VZV) vaccine composition, which can further improve the immunological activity of the varicella-zoster vaccine.

To achieve the above objective, the technical solution of the present disclosure is as follows:
A varicella-zoster virus (VZV) vaccine composition, comprising a VZV gE antigen and a CpG ODN adjuvant, wherein the nucleotide sequence of the CpG ODN adjuvant is set forth in SEQ ID NO: 1.

In an embodiment, at least one nucleotide in the CpG ODN adjuvant is phosphorothioated; preferably, all nucleotides are phosphorothioated.

Further, in the composition, the content of the VZV gE antigen is 20 to150 µg, preferably 40 to 100 µg.

Further, in the composition, the content of the CpG ODN adjuvant is 10 to 3000 µg, preferably 20 to 2000 µg, and more preferably 50 to 500 µg.

It should be noted that regarding the vaccine dosage, the volume per dose is capable of inducing a protective immune response in the human body without significant toxic side effects. In the prior art, the volume per dose for a Varicella-Zoster vaccine is generally 0.5 mL. That is, the aforementioned contents of the VZV gE antigen and the CpG ODN adjuvant are both calculated based on the volume of a single dose (0.5 mL). Naturally, for animal studies-such as experiments involving mice-the dosage is generally scaled to one-tenth of the human dose (conversion may also be performed based on other criteria, such as body surface area ratios). Based on this standard, a single dose for mouse studies is 0.05 mL; in this context, the content per dose includes approximately 2 to 15 µg of VZV gE antigen, 1 to 300 µg of CpG ODN adjuvant, and a subsequent aluminum adjuvant at approximately 0.03 to 0.05 mg.

Further, the composition further comprises anyone or more of an aluminum adjuvant, monophosphoryl lipid A (MPL), a liposome, and QS-21.

In an embodiment, the aluminum adjuvant is anyone or more of aluminum hydroxide, aluminum phosphate, and amorphous aluminum hydroxyphosphate. MPL is a type of lipopolysaccharide and is a dephosphorylated derivative of lipid A (LPS). Lipopolysaccharides are a class of complex molecules present in the outer membrane of bacterial cells and are one of the key components that trigger an immune response during bacterial infection. MPL is a modified form of LPS; through dephosphorylation, the moiety of the original LPS molecule that causes strong inflammatory responses is reduced. This modification enables MPL to retain its capacity to activate the immune system while simultaneously mitigating potential adverse effects thereof. Its primary function is to activate antigen-presenting cells (APCs) and elicit an adaptive immune response of the immune system, thereby enhancing the efficacy of the vaccine. Liposomes are microscopic vesicles encapsulated by a lipid bilayer. These liposomes can envelop and encapsulate drugs, vaccine antigens, or other active ingredients, forming a structure known as a liposomal carrier, which can be used to deliver the drugs or vaccines. In vaccine preparation, liposomes can encapsulate and carry vaccine antigens, thereby enhancing their stability and potentiating the immune response to the antigens. Furthermore, liposomes can also serve as an immunologic adjuvant to help stimulate the immune system. QS-21 is a triterpene saponin extracted from the bark of *Quillaja saponaria* and is typically utilized as an immunological adjuvant in vaccines. It functions by activating antigen-presenting cells (APCs), thereby enhancing the immune response to vaccine antigens; it also assists in promoting the memory response of the immune system, enabling a more effective response to potential future infections. Furthermore, QS-21 is frequently used in combination with other adjuvants and antigens to improve the immunogenicity of the vaccines.

Further, in the composition, the content of the aluminum adjuvant is 300 to 500 µg, and preferably 350 to 450 µg.

Further, in the composition, the content of the VZV gE antigen is preferably 80 to 200 µg/mL (40 to 100 µg), the content of the CpG ODN adjuvant is preferably 100 to 1000 µg/mL (50 to 500 µg), and the content of the aluminum adjuvant is 600 to 1000 µg/mL (300 to 500 µg).

Further, the amino acid sequence of the VZV gE antigen is selected from any one of SEQ ID NOs: 2-6.

The present disclosure further provides a method for preparing the varicella-zoster vaccine composition according to any one of the preceding items, comprising the following steps:
1) Preparing VZV gE protein: synthesizing a gene encoding the VZV gE protein and constructing an expression vector; transfecting the constructed vector into CHO cells; establishing a stable transfected cell pool; screening the pool to identify cell lines that stably express the VZV gE protein; and finally performing protein expression and purification to obtain the VZV gE antigen having an amino acid sequence as set forth in any one of SEQ ID NOs: 2-6; wherein the protein purification typically involves methods such as chromatography, centrifugation, and filtration to isolate the VZV gE protein from a collected cell culture supernatant;
2) Preparing a vaccine composition comprising CpG ODN as the sole adjuvant: prior to injection, adding CpG ODN to the VZV gE protein obtained in step 1) and mixing thoroughly;
3) Preparing a vaccine composition comprising aluminum adjuvant as the sole adjuvant: adding an aluminum adjuvant (Alum) to the VZV gE protein obtained in step 1), mixing thoroughly, and incubating at 4 °C overnight;
4) Preparing a dual-adjuvant vaccine composition: adding an aluminum adjuvant (Alum) to the VZV gE protein obtained in step 1), mixing thoroughly, and incubating at 4 °C overnight; then, prior to injection, adding CpG ODN and mixing thoroughly.

The present disclosure further provides use of the varicella-zoster vaccine composition according to any one of the preceding items in the manufacture of a medicament for preventing and/or treating varicella-zoster virus infection and/or a disease mediated by varicella-zoster virus.

The present disclosure further provides use of the varicella-zoster vaccine composition according to any one of the preceding items in the manufacture of a medicament for eliciting a humoral and/or cellular immune response against varicella-zoster virus in a subject.

Compared with the prior art, the present disclosure offers the following advantages:
In the present disclosure, by selecting a CpG ODN adjuvant with a specific nucleotide sequence, superior immunostimulatory activity is achieved; the dual-adjuvant vaccine, combining the specific CpG ODN and an aluminum adjuvant, can elicit a high humoral immune response at an earlier stage, thereby inducing protective immunity at least one month earlier. A single immunization with the present composition can achieve antibody levels comparable to-or nearly equivalent to-those attained after two doses of the Shingrix vaccine, while also maintaining a stable, high level of humoral immunity over an extended period.

### DESCRIPTION OF THE DRAWINGS

FIG. 1 is a graph showing the detection results of the effects of different doses of CpG HP007 on the immunogenicity of herpes zoster vaccine compositions in Example 2.
FIG. 2 is a graph showing the detection results of the effects of different adjuvants on the immunogenicity (humoral immune response) of herpes zoster vaccine compositions in Example 3.
FIG. 3 is a graph showing the detection results of the effects of different adjuvants on the immunogenicity (cellular immune response) of herpes zoster vaccine compositions in Example 3.
FIG. 4A is a graph showing the immunogenicity detection results comparing the dual-adjuvant herpes zoster vaccine composition containing CpG HP007 with that containing CpG 684 in Example 4.
FIG. 4B is a graph showing the immunogenicity detection results comparing the dual-adjuvant herpes zoster vaccine composition containing CpG HP007 with that containing CpG 7909 in Example 4.
FIG. 5 is a graph showing the immunogenicity detection results of different herpes zoster vaccines in Example 5.

### DETAILED DESCRIPTION OF THE INVENTION

The following description sets forth numerous specific details to facilitate thorough understanding of the present disclosure. However, the present disclosure may be practiced in many ways different from those described herein; those skilled in the art may make similar modifications and improvements without departing from the spirit and scope of the present disclosure. Therefore, the present disclosure is not limited to the specific embodiments disclosed below.

Unless otherwise defined, all technical and scientific terms used in the present disclosure have the same meaning as commonly understood by a person skilled in the art to which this disclosure pertains. The terminology used in the description of the present disclosure herein is for the purpose of describing particular embodiments only and is not intended to be limiting of the disclosure.

### DEFINITIONS

Unless otherwise indicated, the practice of the present disclosure will employ conventional techniques of molecular biology (including recombinant techniques), microbiology, cell biology, biochemistry, and immunology, all of which are within the skill of the art.

The phrase "comprising" as used in the present disclosure is open-ended, indicating that such embodiments may include additional elements. In contrast, the phrase "consisting of" is closed-ended, indicating that such embodiments do not include additional elements (other than trace impurities). The phrase "consisting essentially of" is partially closed-ended, indicating that such embodiments may further include elements that do not materially affect the basic characteristics of such embodiments.

As used herein, the term "about" with respect to a value encompasses 90% to 110% of that value (e.g., "about 100 µg of HP007" refers to 90 µg to 110 µg of HP007).

An "effective amount" or a "sufficient amount" of a substance is an amount sufficient to achieve beneficial or desired results, including clinical results, and as such, an "effective amount" depends on the context in which it is applied. In the context of administering an immunogenic composition, an effective amount contains sufficient antigen and TLR9 agonist to stimulate an immune response (preferably a seroprotective level of antibodies against the antigen).

The terms "individual" and "subject" refer to a mammal. "Mammals" include, but are not limited to, humans, non-human primates (e.g., monkeys), livestocks, sport animals, rodents (e.g., mice and rats), and pets (e.g., dogs and cats).

As used herein, the term "dose" in reference to a vaccine composition refers to a measured portion of the vaccine composition taken (administered or received) by a subject at any one time.

As used herein, the terms "isolated" and "purified" refer to material that is removed from at least one component with which it is naturally associated (e.g., removed from its original environment). When used with respect to a recombinant protein, the term "isolated" refers to said protein that has been removed from the culture medium of the host cells producing the protein.

As used herein, the term "vaccine" refers to any biological product-including, but not limited to, proteins, polysaccharides, nucleic acids, live vectors, or infectious agents-that, upon vaccination via injection or a mucosal route, induces the body to generate specific antibodies and/or cellular immunity against a specific pathogen, thereby enabling the body to acquire protection against or the ability to eliminate said pathogen. Vaccines are active immunizing agents used for the prevention of infectious diseases, produced from pathogenic microorganisms (such as bacteria, rickettsia, viruses, etc.) and their metabolites through processes such as artificial attenuation, inactivation, or genetic engineering. A vaccine retains the characteristics of a pathogen for stimulating an immune system of an animal body. When an animal is exposed to these non-harmful pathogens, its immune system generates specific protective substances such as immune hormones, bioactive physiological substances, and specific antibodies; when the animal is again exposed to said pathogens, the immune system of the animal body follows its original memory to produce an increased amount of protective substances, thereby preventing the pathogens from causing harm.

As used herein, the term "immunization" refers to a process of increasing the response of a mammalian subject to an antigen, and thereby improving its ability to resist or overcome infection and/or resist disease.

As used herein, the term "vaccination" refers to the introduction of a vaccine into the body of a mammalian subject.

The term "adjuvant" refers to a substance that, when added to a composition comprising an antigen, enhances or augments an immune response of a mammalian recipient to the antigen following exposure. Adjuvants include, but are not limited to, alum (aluminum salts), oil-in-water emulsions, water-in-oil emulsions, liposomes, and microparticles such as poly(lactide-co-glycolide) microparticles.

As used interchangeably herein, the terms "polynucleotide" and "oligonucleotide" refer to molecules formed by the linkage of mononucleotides, each of which is composed of a sugar (such as deoxyribose or ribose), a phosphate group, and a base; wherein the sugar molecule and the base are linked to form a nucleoside, and nucleosides are linked via phosphate groups to form nucleotides. The bases constituting the nucleosides include pyrimidines and purines; the pyrimidines include thymine (T or t), cytosine (C or c), uracil (U or u), or derivatives thereof, and the purines include adenine (A or a), guanine (G or g), or derivatives thereof. "Polynucleotides" and "oligonucleotides" include single-stranded DNA (ssDNA), double-stranded DNA (dsDNA), single-stranded RNA (ssRNA), and double-stranded RNA (dsRNA), modified oligonucleotides and oligonucleosides, or combinations thereof. The configuration of an oligonucleotide may be linear or circular, or the oligonucleotide may comprise both linear and circular segments. Oligonucleotides are polymers of nucleosides typically linked via phosphodiester bonds; however, alternative linkages, such as phosphorothioate bonds, may also be employed within the oligonucleotide.

As used herein, the terms "CpG", "CpG motif", and "cytosine-phospho-guanosine" refer to unmethylated cytidine-phospho-guanosine dinucleotides that, when present in an oligonucleotide, contribute to a measurable immune response *in vitro*, *in vivo*, and/or *ex vivo*. Examples of measurable immune responses include, but are not limited to, antigen-specific antibody production, secretion of cytokines, and activation or expansion of lymphocyte populations (such as NK cells, CD4⁺ T lymphocytes, CD8⁺ T lymphocytes, B lymphocytes, etc.). Preferably, the CpG oligonucleotide preferentially activates a Th1-type response.

As used herein, the term "CpG ODN" refers to a synthetic CpG-containing oligodeoxynucleotide capable of enhancing antibody production and stimulating a T helper 1 (Th1) cell response.

The present disclosure is described in detail below with reference to specific examples.

In the following examples, the sources of materials are as follows:
PBS (phosphate-buffered saline) dry powder was purchased from Solarbio;
CpG HP007, CpG 684, and CpG 7909 were provided by Jiangsu Taipuri Biopharmaceutical Technology Co., Ltd.;
Aluminum hydroxide adjuvant was purchased from Thermo Fisher Scientific;
MF59 adjuvant was purchased from AVT (Shanghai) Pharmaceutical Tech Co., Ltd.;
BALB/c mice and C57BL/6J mice were purchased from Vital River Laboratory Animal Technology Co., Ltd.
Specifically, the nucleotide sequence of CpG HP007 is set forth in SEQ ID NO: 1, which is: 5'-tcgcgaacgttcgccgcgtacgtacgcgg-3';
The nucleotide sequence of CpG 684 is set forth in SEQ ID NO: 7, which is: 5'-tcgacgttcgtcgttcgtcgttc-3';
The nucleotide sequence of CpG 7909 is set forth in SEQ ID NO: 8, which is: 5'-tcgtcgttttgtcgttttgtcgtt-3'.

### Example 1: Preparation of Herpes Zoster Vaccine Compositions

In order to investigate the technical effects of the herpes zoster vaccine compositions provided by the present disclosure, a variety of the following herpes zoster vaccine compositions were prepared in this example, each containing VZV gE protein, an aluminum adjuvant, and/or a CpG ODN adjuvant. The specific formulation methods are as follows:
1. Preparation of VZV gE proteins (expressed in CHO cells), comprising the following steps: (1) Molecular cloning: gene synthesis and construction of expression vectors; (2) Establishment of expression cell lines: stable expression cell pools and monoclonal cell lines; (3) Protein expression and purification. The five VZV gE proteins prepared are as follows:
   SEQ ID NO:2 
(MGTVNKPVVGVLMGFGIITGTLRITNPVRASVLRYDDFHIDEDKLDTNSVYEPYY
HSDHAESSWVNRGESSRKAYDHNSPYIWPRNDYDGFLENAHEHHGVYNQGRGIDSGER
LMQPTQMSAQEDLGDDTGIHVIPTLNGDDRHKIVNVDQRQYGDVFKGDLNPKPQGQRL
IEVSVEENHPFTLRAPIQRIYGVRYTETWSFLPSLTCTGDAAPAIQHICLKHTTCFQDVVV
DVDCAENTKEDQLAEISYRFQGKKEADQPWIVVNTSTLFDELELDPPEIEPGVLKVLRTE
KQYLGVYIWNMRGSDGTSTYATFLVTWKGDEKTRNPTPAVTPQPRGAEFHMWNYHSH
VFSVGDTFSLAMHLQYKIHEAPFDLLLEWLYVPIDPTCQPMRLYSTCLYHPNAPQCLSH
MNSGCTFTSPHLAQRVASTVYQNCEHADNYTAYCLGISHMEPSFGLILHDGGTTLKFVD
TPESLSGLYVFVVYFNGHVEAVAYTVVSTVDHFVNAIEERGFPPTAGQPPATTKPKEITP
VNPGTSPLIRYAAWTGGLA),
   SEQ ID NO:3 
(MGTVNKPVVGVLMGFGIITGTLRITNPVRASVLRYDDFHTDEDKLDTNSVYEPYY
HSDHAESSWVNRGESSRKAYDHNSPYIWPRNDYDGFLENAHEHHGVYNQGRGIDSGER
LMQPTQMSAQEDLGDDTGIHVIPTLNGDDRHKIVNVDQRQYGDVFKGDLNPKPQGQRL
IEVSVEENHPFTLRAPIQRIYGVRYTETWSFLPSLTCTGDAAPAIQHICLKHTTCFQDVVV
DVDCAENTKEDQLAEISYRFQGKKEADQPWIVVNTSTLFDELELDPPEIEPGVLKVLRTE
KQYLGVYIWNMRGSDGTSTYATFLVTWKGDEKTRNPTPAVTPQPRGAEFHMWNYHSH
VFSVGDTFSLAMHLQYKIHEAPFDLLLEWLYVPIDPTCQPMRLYSTCLYHPNAPQCLSH
MNSGCTFTSPHLAQRVASTVYQNCEHADNYTAYCLGISHMEPSFGLILHDGGTTLKFVD
TPESLSGLYVFVVYFNGHVEAVAYTVVSTVDHFVNAIEERGFPPTAGQPPATTKPKEITP
VNPGTSPLLRYAA),
   SEQ ID NO:4 
(MGTVNKPVVGVLMGFGIITGTLRITNPVRASVLRYDDFHTDEDKLDTNSVYEPYY
HSDHAESSWVNRGESSRKAYDHNSPYIWPRNDYDGFLENAHEHHGVYNQGRGIDSGER
LMQPTQMSAQEDLGDDTGIHVIPTLNGDDRHKIVNVDQRQYGDVFKGDLNPKPQGQRL
IEVSVEENHPFTLRAPIQRIYGVRYTETWSFLPSLTCTGDAAPAIQHICLKHTTCFQDVVV
DVDCAENTKEDQLAEISYRFQGKKEADQPWIVVNTSTLFDELELDPPEIEPGVLKVLRTE
KQYLGVYIWNMRGSDGTSTYATFLVTWKGDEKTRNPTPAVTPQPRGAEFHMWNYHSH
VFSVGDTFSLAMHLQYKIHEAPFDLLLEWLYVPIDPTCQPMRLYSTCLYHPNAPQCLSH
MNSGCTFTSPHLAQRVASTVYQNCEHADNYTAYCLGISHMEPSFGLILHDGGTTLKFVD
TPESLSGLYVFVVYFNGHVEAVAYTVVSTVDHF),
   SEQ ID NO:5 
(MGTVNKPVVGVLMGFGIITGTLRITNPVRASVLRYDDFHTDEDKLDTNSVYEPYY
HSDHAESSWVNRGESSRKAYDHNSPYIWPRNDYDGFLENAHEHHGVYNQGRGIDSGER
LMQPTQMSAQEDLGDDTGIHVIPTLNGDDRHKIVNVDQRQYGDVFKGDLNPKPQGQRL
IEVSVEENHPFTLRAPIQRIYGVRYTETWSFLPSLTCTGDAAPAIQHICLKHTTCFQDVVV
DVDCAENTKEDQLAEISYRFQGKKEADQPWIVVNTSTLFDELELDPPEIEPGVLKVLRTE
KQYLGVYIWNMRGSDGTSTYATFLVTWKGDEKTRNPTPAVTPQPRGAEFHMWNYHSH
V),
   SEQ ID NO:6 
(MGTVNKPVVGVLMGFGIITGTLRITNPVRASVLRYDDFHIDEDKLDTNSVYEPYY
HSDHAESSWVNRGESSRKAYDHNSPYIWPRNDYDGFLENAHEHHGVYNQGRGIDSGER
LMQPTQMSAQEDLGDDTGIHVIPTLNGDDRHKIVNVDQRQYGDVFKGDLNPKPQGQRL
 
IEVSVEENHPFTLRAPIQRIYGVRYTETWSFLPSLTCTGDAAPAIQHICLKHTTCFQDVVV
DVDCAENTKEDQLAEISYRFQGKKEADQPWIVVNTSTLFDELELDPPEIEPGVLKVLRTE
KQYLGVYIWNMRGSDGTSTYATFLVTWKGDEKTRNPTPAVTPQPRGAEFHMWNYHSH
VFSVGDTFSLAMHLQYKIHEAPFDLLLEWLYVPIDPTCQPMRLYSTCLYHPNAPQCLSH
MNSGCTFTSPHLAQRVASTVYQNCEHADNYTAYCLGISHMEPSFGLILHDGGTTLKFVD
TPESLSGLYVFVVYFNGHVEAVAYTVVSTVDHFVNAIEERGFPPTAGQPPATTKPKEITP
VNPGTSPLLRYAAWTGGLA).
2. Preparation of a vaccine composition comprising CpG ODN as the sole adjuvant: Prior to injection, CpG ODN was added to the VZV gE protein obtained in Step 1 and mixed thoroughly.
3. Preparation of a vaccine composition comprising aluminum adjuvant as the sole adjuvant: Aluminum adjuvant (Alum) was added to the VZV gE protein obtained in Step 1, mixed thoroughly, and incubated at 4 °C overnight.
4. Preparation of a multi-adjuvant vaccine composition: Aluminum adjuvant (Alum) was added to the VZV gE protein obtained in Step 1, mixed thoroughly, and incubated at 4 °C overnight. Then, prior to injection, CpG ODN was added and mixed thoroughly.

Example 2: Effect of CpG HP007 Dosage on the Immunogenicity of Herpes Zoster Vaccine Compositions in Mice

In order to evaluate the effect of CpG HP007 dosage on the immunogenicity of the herpes zoster vaccine compositions prepared in Example 1, an immunogenicity study was conducted in this example using C57BL/6J mice as an animal model. The specific methods for evaluating immunogenicity are as follows:

### 1. Animal Experimental Design

Eight-week-old female C57BL/6J mice were randomly assigned to 7 groups, with 12 mice per group. On Day 0 (D0) and Day 28 (D28), mice in each group were immunized via intramuscular injection with different herpes zoster vaccine compositions at an injection volume of 50 µL. Retro-orbital blood collection was performed on Day 21 (D21), Day 28 (D28, prior to the second immunization), and Day 31 (D31), with 200 to 300 µL of blood collected from each mouse at each time point. Serum was separated and binding antibody titers were determined via ELISA. The grouping of mice in this example is shown in Table 1.

**Table 1: Grouping of experimental mice**

| Group | Vaccine Components and Dosages |
|---|---|
| 1 | PBS |
| 2 | 5 µg gE |
| 3 | 5 µg gE + 1 µg HP007 |
| 4 | 5 µg gE + 8 µg HP007 |
| 5 | 5 µg gE + 24 µg HP007 |
| 6 | 5 µg gE + 72 µg HP007 |
| 7 | 5 µg gE + 8 µg HP007 + 40 µg Alum |

Specifically, gE refers to the VZV gE antigen, taking the sequence set forth in SEQ ID NO: 2 prepared in Example 1 as an example; HP007 refers to the CpG HP007 with the sequence set forth in SEQ ID NO: 1.

### 2. Determination of Binding Antibody Titers

The titers of gE protein-specific binding antibodies in mouse serum collected on D21, D28 (prior to the second immunization), and D31 were determined via ELISA.
(1) Assay Method: The antigen gE protein (CHO) was coated onto a microtiter plate to capture gE protein-specific antibodies in mouse serum. Unbound sites were blocked with a blocking buffer, followed by the addition of diluted mouse serum, blank controls, and standards. Subsequently, horseradish peroxidase (HRP)-conjugated goat anti-mouse IgG secondary antibody was added to bind the antibodies in the mouse serum to be tested. A chromogenic solution was added for color development, and the reaction was stopped by adding a stop solution. The color reaction was observed, and the absorbance at 450 nm (A450) was detected using a microplate reader.
(2) Result Determination: The cut-off value (COV) was defined according to the following formula: $COV = 2.1 \times {OD}_{blank}$

OD_{blank} represents the OD value of the blank control group.

The antibody titer was defined as the highest serum dilution that resulted in an absorbance value greater than the COV. Antibody potency was expressed as the Geometric Mean Titer (GMT).

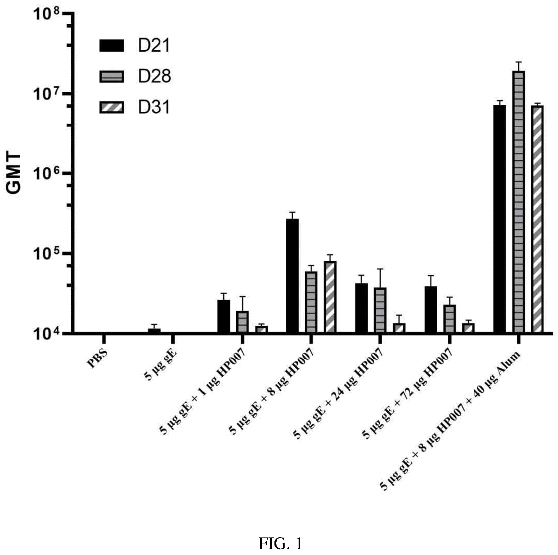

(3) The detection results are shown in Figure 1.

As illustrated in Figure 1, CpG HP007 was able to enhance the immunogenicity of the antigen and exhibited a dose-dependent relationship within the dose range of 1 to 72 µg. Furthermore, compared with the vaccine group using CpG HP007 alone, the dual-adjuvant vaccine group (CpG HP007 + Alum) significantly enhanced VZV gE-specific antibody titers (by more than 100-fold).

Example 3: Comparison of Immunogenicity of Herpes Zoster Vaccine Compositions Comprising Different Adjuvants in Mice

To evaluate the effects of different adjuvants on the immunogenicity of herpes zoster vaccine compositions, an immunogenicity study was conducted in this example using C57BL/6J mice as an animal model. The specific methods for evaluating immunogenicity are as follows:

### 1. Animal Experimental Design

Eight-week-old female C57BL/6J mice were randomly assigned to 6 groups, with 12 mice per group. On D0 and D28, mice in each group were immunized via intramuscular injection with different herpes zoster vaccine compositions at an injection volume of 50 µL. Retro-orbital blood collection was performed on D21, D28 (prior to the second immunization), and D31, with 200 to 300 µL of blood collected from each mouse at each time point. Serum was separated and binding antibody titers were determined via ELISA. On D35, a subset of mice from Groups 1, 3, 4, and 6 were euthanized; spleen tissues were harvested, and splenic lymphocytes were isolated for ELISPOT analysis. The grouping of mice in this example is shown in Table 2.

**Table 2: Grouping of experimental mice**

| Group | Vaccine Components and Dosages |
|---|---|
| 1 | PBS |
| 2 | 5 µg gE |
| 3 | 5 µg gE + 8 µg HP007 |
| 4 | Shingrix |
| 5 | 5 µg gE + MF59 |
| 6 | 5 µg gE + 8 µg HP007 + 40 µg Alum |

Specifically, in Group 4, each mouse was vaccinated with commercial Shingrix (GSK), containing 5 µg of gE protein and 50 µL of AS01_{B} adjuvant. Except for Shingrix, which is a commercially available herpes zoster vaccine, the VZV gE proteins used in all other groups were self-produced gE proteins. Specifically, the VZV gE antigen prepared in Example 1, having the sequence set forth in SEQ ID NO: 3, was used as an example. HP007 refers to the CpG HP007 having the sequence set forth in SEQ ID NO: 1. Shingrix is a recombinant herpes zoster vaccine known in the prior art that employs the novel adjuvant AS01_{B} (MPL + QS-21). MF59 is also a vaccine adjuvant commonly used in the prior art, the primary components of which include an oil phase, an aqueous phase, and surfactants.

### 2. Determination of Binding Antibody Titers

Binding antibody titers specific to the gE protein in mouse serum collected on D21, D28 (prior to the second immunization), and D31 were determined via ELISA.

The assay methods and result interpretation criteria were as described in Example 2.

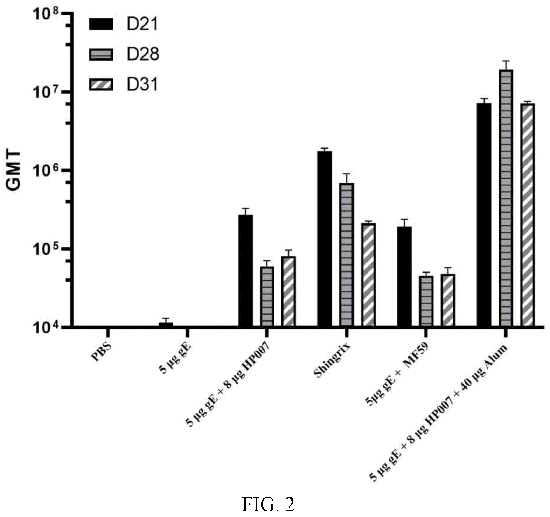

The detection results are shown in Figure 2.

As illustrated in Figure 2, on D21 and D28 following the first immunization, the antibody titers in the CpG HP007 + Alum dual-adjuvant vaccine group were 10-fold or more higher than those in the Shingrix group. Across the serum samples at these three time points, the antibody titers in the CpG HP007 + Alum group remained at a consistently high level, exhibiting no significant changes.

These results indicate that the CpG HP007 + Alum dual-adjuvant vaccine elicits a high humoral immune response at an earlier stage, thereby inducing protective immunity at least one month earlier. Furthermore, a single vaccination of the CpG HP007 + Alum dual-adjuvant vaccine achieves antibody levels comparable to-or nearly equivalent to-those attained after two vaccinations of the Shingrix vaccine, while maintaining consistently high levels of humoral immunity over an extended period.

### 3. Detection of Cellular Immune Responses

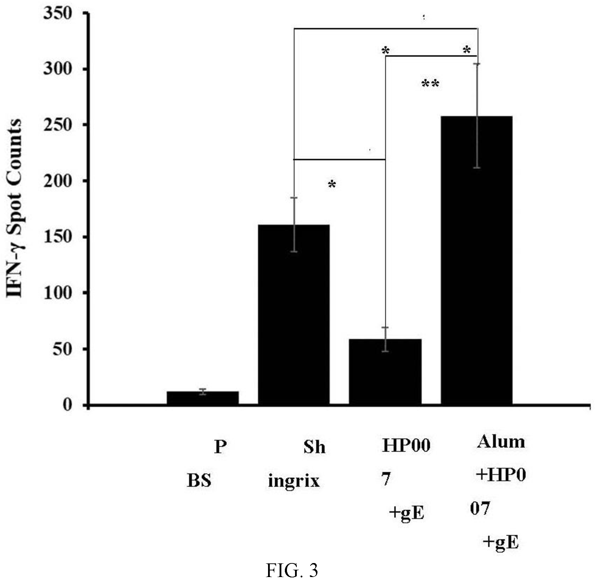

gE-specific T cells in the mouse spleen were quantified via ELISPOT. Specifically, the number of IFN-γ-secreting splenocytes was determined using an IFN-γ ELISPOT assay (IFN-γ ELISPOT Kit, BD Biosciences).
(1) Assay Method: Splenocytes from immunized mice were seeded into 96-well plates at a density of 3×10⁵ cells/well. gE protein was added to a final concentration of 5 µg/mL, with culture medium serving as a negative control. The cells were then incubated at 37 °C in a 5% CO₂ atmosphere for 24 hours. The supernatant was discarded, and after washing, a detection antibody (biotinylated anti-IFN-γ antibody) was added, followed by incubation at room temperature for 2 hours. After washing, Streptavidin-HRP was added and incubated at room temperature for 1 hour. After washing, a substrate solution was added for color development. The reaction was terminated with deionized water, and the plates were dried. Spots were counted using an ELISPOT reader.
(2) The detection results are shown in Figure 3.

Figure 3 presents the ELISpot assay results for D35. As indicated in Figure 3, the levels of gE-specific cellular immunity in the HP007 + Alum dual-adjuvant vaccine group were significantly higher than those in the Shingrix group and the HP007 single-adjuvant vaccine group (*P < 0.01*).

Conclusion: The HP007 + Alum dual-adjuvant vaccine is capable of eliciting a robust cellular immune response, demonstrating significant superiority over Shingrix.

Example 4: Comparison of the Effects of Different CpG ODNs on the Immunogenicity of Herpes Zoster Vaccines

To evaluate the effects of different adjuvants on the immunogenicity of herpes zoster vaccine compositions, an immunogenicity study was conducted in this example using BALB/c mice as an animal model. The methods for evaluating immunogenicity are detailed as follows:

### 1. Animal Experimental Design

Eight-week-old BALB/c mice were randomly assigned into 9 groups, with 8 mice per group. On D0, each group was immunized via intramuscular injection with a dose of 0.1 mL per mouse. On D7, blood samples were collected via the tail vein, and serum was separated. Anti-gE antibody titers in the mouse serum were determined via ELISA by measuring OD₄₅₀ values, following the same methodology described above. The specific grouping for this example is detailed in Table 3.

**Table 3: Grouping of experimental mice**

| Group | Vaccine Components and Dosages |
|---|---|
| 1 | PBS |
| 2 | 10 µg gE |
| 3 | 10 µg gE + 40 µg Al(OH)₃ |
| 4 | 2.5 µg CpG 684 + 10 µg gE + 40 µg Al(OH)₃ |
| 5 | 5 µg CpG 684 + 10 µg gE + 40 µg Al(OH)₃ |
| 6 | 2.5 µg CpG 7909 + 10 µg gE + 40 µg Al(OH)₃ |
| 7 | 5 µg CpG 7909 + 10 µg gE + 40 µg Al(OH)₃ |
| 8 | 2.5 µg CpG HP007 + 10 µg gE + 40 µg Al(OH)₃ |
| 9 | 5 µg CpG HP007 + 10 µg gE + 40 µg Al(OH)₃ |

Specifically, the gE antigen is the VZV gE antigen prepared in Example 1, having the sequence set forth in SEQ ID NO: 4, as an illustrative example. The sequence of CpG 684 is set forth in SEQ ID NO: 7; the sequence of CpG 7909 is set forth in SEQ ID NO: 8; and the sequence of CpG HP007 is set forth in SEQ ID NO: 1.

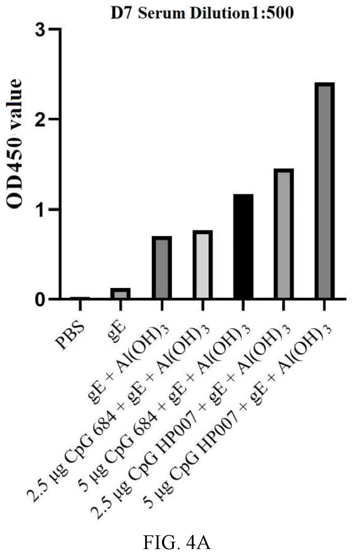

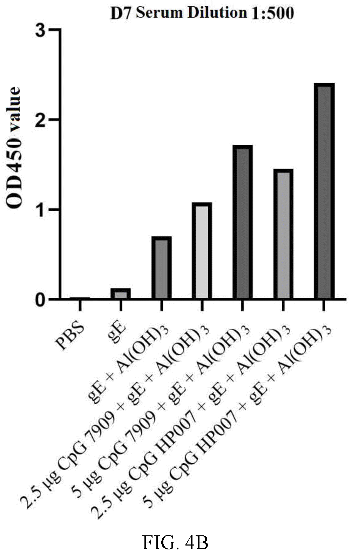

2. The detection results are shown in Figures 4A and 4B.

As shown in Figure 4A, the dual-adjuvant vaccine groups significantly increased anti-gE antibody titers compared to the aluminum-adjuvant-only group. Furthermore, antibody titers in the CpG HP007 + aluminum hydroxide dual-adjuvant vaccine group were higher than those in the CpG 684 + aluminum hydroxide dual-adjuvant vaccine group, suggesting that CpG HP007 exhibits superior immunostimulatory activity to CpG 684 within the context of herpes zoster vaccines.

As shown in Figure 4B, the dual-adjuvant vaccine groups significantly increased anti-gE antibody titers compared to the aluminum-adjuvant-only group. Furthermore, the antibody titers in the CpG HP007 + aluminum hydroxide dual-adjuvant vaccine group were higher than those in the CpG 7909 + aluminum hydroxide dual-adjuvant vaccine group, demonstrating that CpG HP007 possesses superior immunostimulatory activity compared to CpG 7909 in the context of herpes zoster vaccines.

### Example 5: Comparison of the Immunogenicity of Different Herpes Zoster Vaccines

To compare the immunogenicity of different herpes zoster vaccine compositions, an immunogenicity study was conducted in this experiment using C57BL/6J mice as an animal model. The methods for evaluating immunogenicity are detailed as follows:

### 1. Animal Experimental Design

Eight-week-old female C57BL/6J mice were randomly assigned to 5 groups, with 12 mice per group. On D0 and D28, mice in each group were immunized via intramuscular injection with different herpes zoster vaccine compositions at an injection volume of 50 µL. Retro-orbital blood collection was performed on D21, D28 (prior to the second immunization), D31, and D56, with 200 to 300 µL of blood collected from each mouse at each time point. Serum was separated and binding antibody titers were determined via ELISA. The grouping of mice in this example is shown in Table 4.

**Table 4: Grouping of experimental mice**

| Group | Vaccine Components and Dosages |
|---|---|
| 1 | PBS |
| 2 | 5 µg gE |
| 3 | 10 µg gE + 40 µg Alum + 5 µg CpG 684 |
| 4 | 5 µg gE + 40 µg Alum + 8 µg CpG HP007 |
| 5 | 5 µg gE + 40 µg Alum + 5 µg CpG HP007 |

Specifically, the gE antigen used herein is the VZV gE antigen prepared in Example 1, having the sequence set forth in SEQ ID NO: 5 as an illustrative example.

### 2. Determination of Binding Antibody Titers

ELISA was employed to determine the gE-specific antibody titers in mouse serum collected on D21, D28 (prior to the second immunization), D31, and D56. The assay methodology and criteria for result determination were as described in Example 2.

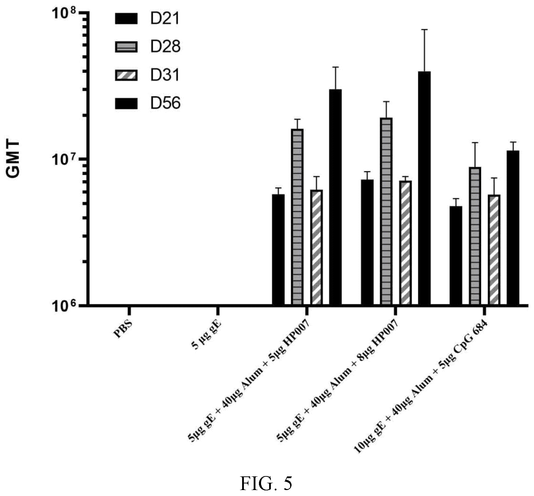

The detection results are shown in Figure 5.

As indicated in Figure 5, the immunogenicity of the vaccine group containing 5 µg gE + 40 µg Alum + 8 µg CpG HP007 was superior to that of the vaccine group containing 10 µg gE + 40 µg Alum + 5 µg CpG 684, thereby demonstrating the superiority of the former vaccine composition in terms of both components and their ratios.

The technical features of the present disclosure may be combined in any manner. For the sake of conciseness, not all possible combinations of the technical features in the embodiments are described. However, as long as there is no contradiction in the combination of these technical features, such combinations should be considered within the scope of the present disclosure.

The above-described embodiments merely represent several embodiments of the present disclosure, and the descriptions thereof are specific and detailed, but they should not be construed as limiting the scope of the present disclosure. It should be noted that for those of ordinary skill in the art, some modifications and improvements may be made without departing from the concept of the present disclosure, all of which fall within the protection scope of the present disclosure. Therefore, the protection scope of the present disclosure shall be defined by the appended claims.

## Claims

1. A varicella-zoster virus (VZV) vaccine composition, comprising a VZV gE antigen and a CpG ODN adjuvant, wherein the nucleotide sequence of the CpG ODN adjuvant is set forth in SEQ ID NO: 1.

2. The VZV vaccine composition according to claim 1, wherein the content of the VZV gE antigen in the composition is 20 to 150 µg.

3. The VZV vaccine composition according to claim 1, wherein the content of the CpG ODN adjuvant in the composition is 10 to 3000 µg.

4. The VZV vaccine composition according to claim 1, further comprising anyone or more of an aluminum adjuvant, MPL, a liposome, and QS-21.

5. The VZV vaccine composition according to claim 4, wherein the content of the aluminum adjuvant in the composition is 300 to 500 µg.

6. The VZV vaccine composition according to claim 4, wherein the aluminum adjuvant is anyone or more of aluminum hydroxide, aluminum phosphate, and amorphous aluminum hydroxyphosphate.

7. The VZV vaccine composition according to claim 1, wherein the amino acid sequence of the VZV gE antigen is selected from any one of SEQ ID NOs: 2-6.

8. Use of the VZV composition according to any one of claims 1 to 7 in the manufacture of a medicament for preventing and/or treating varicella-zoster virus infection and/or a disease mediated by varicella-zoster virus.

9. Use of the VZV composition according to any one of claims 1 to 7 in the manufacture of a medicament for eliciting a humoral and/or cellular immune response against varicella-zoster virus in a subject.
